(19) **Europäisches Patentamt / European Patent Office / Office européen des brevets**

(11) **EP 2 872 187 B1**

(12) **FASCICULE DE BREVET EUROPEEN**

(45) Date de publication et mention
de la délivrance du brevet:
**26.12.2018 Bulletin 2018/52**

(51) Int Cl.:
*A61L 15/28* (2006.01)    *A61L 15/44* (2006.01)
*A61Q 19/06* (2006.01)

(21) Numéro de dépôt: **13735301.7**

(86) Numéro de dépôt international:
**PCT/EP2013/064718**

(22) Date de dépôt: **11.07.2013**

(87) Numéro de publication internationale:
**WO 2014/009488 (16.01.2014 Gazette 2014/03)**

(54) **PANSEMENT A LIBERATION PROLONGEE D'ACTIFS**

WUNDVERBAND MIT GESTEUERTER FREISETZUNG VON WIRKSTOFFEN

DRESSING HAVING THE CONTROLLED RELEASE OF ACTIVE AGENTS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **13.07.2012  FR 1256829**

(43) Date de publication de la demande:
**20.05.2015  Bulletin 2015/21**

(73) Titulaire: **Urgo Recherche Innovation et
Développement
21300 Chenove (FR)**

(72) Inventeurs:
• **DESMAISON, Nadège**
  **F-21110 Tart le Haut (FR)**
• **RUAULT, Aurélie**
  **F-21000 Dijon (FR)**
• **AUGUSTE, Stéphane**
  **F-21490 Ruffey les Echirez (FR)**

(74) Mandataire: **Hirsch & Associés
137, rue de l'Université
75007 Paris (FR)**

(56) Documents cités:
**FR-A1- 2 956 322**

## Description

La présente invention concerne de nouveaux pansements à base d'oligosaccharides polysulfatés présentant une libération prolongée desdits principes actifs. Elle concerne également un procédé pour leur préparation, ce procédé comportant une étape de traitement à l'oxyde d'éthylène. Elle concerne encore leurs utilisations pour le soin des plaies et le traitement et/ou la prévention des cicatrices et des vergetures.

## Etat de la technique antérieure

Les oligosaccharides sont des glucides dont l'hydrolyse fournit uniquement des oses. Ce sont des sucres constitués par l'union d'au moins deux molécules de sucres simples (ou oses). Parmi les oligosaccharides, on trouve le sucrose, également nommé saccharose, sucre double formé par la condensation de 2 oses : une molécule de glucose et une molécule de fructose.

Divers composés oligosaccharidiques sulfatés sont connus dans la littérature et possèdent de multiples activités biologiques, cosmétiques et/ou thérapeutiques. On trouve notamment parmi ces composés, différents sels de sucrose octasulfate tels que le sel de potassium de sucrose octasulfate, le sel de sodium de sucrose octasulfate, le complexe d'hydroxyaluminium de sucrose octasulfate, ou encore les sels d'acides aminés de sucrose octasulfate.

Ces derniers composés sont connus pour leurs actions bénéfiques notamment sur les problèmes d'inflammation gastrique, mais aussi sur la cicatrisation.

La cicatrisation d'une plaie est un phénomène biologique naturel, les tissus humains et animaux étant capables de réparer des lésions localisées par des processus de réparation et de régénération qui leur sont propres.

La cicatrisation naturelle d'une plaie se déroule principalement selon 3 phases successives possédant chacune une activité cellulaire et moléculaire propre. Ainsi, il est retrouvé successivement :
La phase inflammatoire qui débute consécutivement au traumatisme par la mise en oeuvre de phénomènes inflammatoires et vasculaires, telle la formation d'un caillot sanguin composé entre autres de fibrine, médiée par différents facteurs cellulaires et moléculaires, et formant une matrice provisoire appelée tissu « fibrineux » ou tissu « jaune ».

La phase de granulation qui est caractérisée par l'arrivée sur le site de la blessure des fibroblastes et de nouvelles cellules endothéliales nécessaires à la néovascularisation du tissu lésé. Une fois activés, les fibroblastes se transforment en myofibroblastes et participent ainsi à la maturation du tissu de granulation.

La phase d'épithélialisation qui est caractérisée par la réorganisation de la matrice extra-cellulaire. Par exemple, le collagène de type 3 est remplacé par le collagène de type 1. On observe une prolifération de la plupart des cellules. Celles-ci ont un comportement invasif dans un premier temps, type les myofibroblastes, les fibroblastes et les cellules endothéliales, puis voient leur activité diminuer sensiblement. Cette phase permet d'aboutir à terme à une cicatrice remodelée, souple et ne provoquant plus de ressenti douloureux, dans le cadre toutefois d'un processus cicatriciel normal de la plaie. Il arrive cependant parfois qu'à cette étape des cicatrices pathologiques apparaissent, dues à une mauvaise réalisation des étapes terminales de la cicatrisation.

Plusieurs problématiques générales liées au processus cicatriciel, ou à la cicatrice ont déjà fait l'objet d'études, notamment par la Demanderesse.

Un des premiers problèmes rencontré a été celui de l'élimination des tissus nécrotiques et/ou fibrineux lors de la phase inflammatoire. En effet, lorsque le processus de détersion naturelle qui consiste à éliminer ces tissus apparaît insuffisant, le processus de cicatrisation s'en trouve atteint. De nombreuses solutions ont été proposées par le passé, telles que les détersions assistées, du type mécanique ou chirurgicale, enzymatique, autolytique ou encore biologique. Elles ont toutes pour but d'arriver à nettoyer les plaies des tissus fibrineux et nécrotiques qui les composent.

Ces différentes techniques présentent cependant de nombreux désavantages. Elles s'avèrent soit trop douloureuses pour le patient soit trop peu efficaces.

Ces problèmes ont été résolus grâce à l'action de composés décrits dans la demande FR 2 956 322 au nom des Laboratoires URGO. Ce document décrit l'utilisation d'un composé choisi parmi les oligosaccharides polysulfatés ayant 1 à 4 unités d'oses, leurs sels ou leurs complexes, comme agent de détersion d'une plaie.

Parmi ces composés, le sel de potassium du sucrose octasulfate était auparavant connu pour le traitement des plaies lors de la phase de bourgeonnement grâce à son action sur les fibroblastes. Cette action est par exemple décrite dans les demandes de brevet EP 230 023, WO 89/05645 ou WO 98/22114. Ce composé était utilisé après avoir réalisé une détersion assistée de la plaie et donc après avoir éliminé les tissus nécrotiques et/ou fibrineux. On l'utilisait donc sur une plaie propre et détergée.

L'action bénéfique de certains composés de la famille des oligosaccharides polysulfatés en cicatrisation a aussi été décrite. Ainsi, les demandes FR 2 824 474 et FR 2 953 522 décrivent des compositions à base de sucralfate seul ou associé à des sels de métaux de transition pour leur utilisation dans la cicatrisation, la régénération, ou encore la résolution de problèmes d'inflammation de la peau.

Un autre problème que l'on a cherché à résoudre concerne les cicatrices pathologiques et les vergetures.

Les cicatrices pathologiques s'entendent au sens de cicatrices atrophiques, rétractiles ou encore hypertrophiques.

Les vergetures apparaissent à la suite d'un étirement rapide et brutal de la peau. Un tel étirement peut être la conséquence d'un gain de poids et/ou d'une modification hormonale. Chaque vergeture ressemble à une déchirure de la peau. En réalité, c'est le tissu dermique qui est altéré, par un phénomène de transformation des fibroblastes en myofibroblastes. Les vergetures (striae distensae) forment des stries cutanées parallèles et longilignes, de plusieurs centimètres de longueur et de jusqu'à 1 centimètre de largeur. Les vergetures sont parfois fines et peu apparentes, mais elles peuvent présenter de petites dépressions qui donnent à la peau un aspect irrégulier. Dans une première phase, elles varient initialement du rose pâle au rouge violacé (vergetures immatures ou inflammatoires). Au cours du temps, elles ont tendance à changer de couleur et à prendre un aspect blanc nacré (vergetures matures). Elles deviennent alors moins visibles, mais la cicatrice demeure. Les modifications hormonales associées à la prise de poids entraînent l'apparition de vergetures chez de nombreuses femmes au cours de la grossesse. Les facteurs génétiques ont également une influence sur leur apparition. Les vergetures peuvent également apparaître en parallèle de certains états physiologiques ou pathologiques et peuvent constituer un symptôme révélateur d'une maladie génétique. Les principaux facteurs déclenchants sont l'inflammation, le stress mécanique et l'environnement hormonal. L'ensemble de ces facteurs provoque un étirement, une désorientation et une désorganisation des fibres de collagène et d'élastine, sans rupture du tissu de soutien. Les vergetures sont assimilables à des cicatrices (car elles ont subi les mêmes étapes de formation qu'après un traumatisme de la peau). Leur guérison est actuellement impossible, mais une atténuation et une amélioration des lésions sont possibles. Les traitements curatifs sont essentiellement locaux : traitements topiques avec des dérivés de l'acide rétinoïque ou des acides de fruits, emploi de peeling ou de laser. Toutefois, les traitements connus actuellement ne sont pas parfaitement satisfaisants, notamment ils ne sont pas toujours bien tolérés et leur efficacité n'est pas totalement satisfaisante. Il existe une demande pour l'élaboration d'un produit permettant de prévenir et/ou de traiter efficacement les vergetures, avec une tolérance cutanée acceptable.

On connait, notamment au travers de la demande FR 2 956 322, des pansements comprenant un composé choisi parmi le groupe constitué des oligosaccharides polysulfatés possédant de 1 à 4 oses, leurs sels ou leurs dérivés, compris de préférence dans la masse d'enduction ou d'imprégnation du pansement, afin de garantir une biodisponibilité efficace du principe actif, sur le site cicatriciel du patient.

Ces pansements permettent de garantir une biodisponibilité prolongée du principe actif pour prévenir ou traiter les problèmes de détersion, de cicatrisation, les vergetures, ou encore les cicatrices pathologiques. Toutefois, on a constaté qu'après quelques heures d'application, le relargage de principe actif devenait insuffisant. En outre, une partie seulement du principe actif contenu dans le pansement est relargué sur la peau à traiter. Et après quelques heures d'application un nouveau pansement doit être appliqué si l'on souhaite maintenir sur la peau un niveau de principe actif suffisant pour l'efficacité attendue. Outre le coût directement dérivé, un changement régulier de pansement n'est pas toujours compatible avec les activités des utilisateurs/patients. Afin de présenter une meilleure efficacité de traitement, on a cherché à mettre au point des pansements possédant une biodisponibilité prolongée en principe actif sensiblement supérieure. En d'autres termes, on a réalisé des pansements présentant une concentration efficace élevée en principe actif au niveau du site de la lésion, cette efficacité étant prolongée dans le temps. Ce résultat est la conséquence d'une libération ou d'un relargage lui aussi prolongé et élevé de principe actif contenu dans le pansement. Ces nouveaux pansements permettent ainsi un traitement plus rapide et plus efficace du patient.

Par ailleurs, on connaît de l'art antérieur un procédé de stérilisation de pansements par traitement à l'oxyde d'éthylène. Toutefois, ce type de traitement n'a jamais été jusqu'ici appliqué à des pansements à base d'oligosaccharides polysulfatés. En outre, il n'est ni mentionné ni suggéré dans l'art antérieur qu'un tel traitement permette d'allonger la durée de relargage d'un principe actif et d'augmenter la quantité totale de principe actif relargué, bien au contraire. L'art antérieur révèle que tous les procédés de stérilisation (par exemple, la radiostérilisation, la stérilisation par autoclave, ou même la stérilisation à l'oxyde d'éthylène) ont a priori un impact défavorable quant au relargage de principe actif contenu dans un pansement objet d'un tel traitement, soit parce que le type de stérilisation dégrade le principe actif lui-même, soit parce qu'il modifie les propriétés rhéologiques et/ou structurales de la masse élastomérique micro-adhérente, dans laquelle ou sur laquelle est incorporée le ou les principes actifs (Radiation effects on polypropylene/polybutylene blends, Richard J. Rolando, June 1993 Tappi Journal, Vol. 76, N°6 et Influence of processing conditions on medical material degradation/failure, Michael T. K. Ling et al., Antec 200 pages 2724 à 2730).

## Résumé de l'invention

Selon un mode de réalisation préféré, la présente invention consiste en un pansement comprenant au moins une interface micro-adhérente, ladite interface micro-adhérente comprenant au moins un composé choisi parmi les oligosaccharides polysulfatés comprenant 1 à 4 oses, leurs sels et leurs complexes, ledit pansement ayant subi un traitement par de l'oxyde d'éthylène.

Selon un mode de mise en oeuvre préféré, le composé oligosaccharidique polysulfaté est choisi parmi

- le sel de potassium du sucrose octasulfate,
- le sel d'argent du sucrose octasulfate,
- le complexe d'hydroxyaluminium du sucrose octasulfate.

Selon un mode de mise en oeuvre préféré, le pansement comprend de 0,5 à 2 mg/cm2, préférentiellement de 0,7 à 1,9 mg/cm2, avantageusement de 0,9 à 1,7 mg/cm2 de composé choisi parmi les oligosaccharides polysulfatés de 1 à 4 oses, leurs sels et leurs complexes.

Selon un mode de mise en oeuvre préféré, la structure interface micro-adhérente est une composition adhésive élastomérique.

Selon un mode de mise en oeuvre préféré, l'interface micro-adhérente comprend :

- 10 à 60 % en poids d'au moins une résine tackifiante,
- 2 à 20% en poids, de préférence 12 à 16 % en poids d'au moins un composé hydrocolloïde,
- 10 à 65 % en poids d'au moins une huile minérale plastifiante,
- 3 à 25 % en poids d'au moins un polymère élastomérique.

De façon préférée, la couche de masse adhésive élastomérique comprend :

- 0,05 à 1% en poids d'au moins un agent antioxydant,
- 10 à 60 % en poids d'au moins une résine tackifiante,
- 2 à 20% en poids, de préférence 12 à 16 % en poids d'au moins un composé hydrocolloïde,
- 10 à 65 % en poids d'au moins une huile minérale plastifiante,
- 3 à 25 % en poids d'au moins un polymère élastomérique,
- 1 à 15 % en poids d'au moins un agent stabilisant.

Selon un mode de mise en oeuvre préféré, le pansement comprend de 1 à 15% en poids, et de préférence de 5 à 10% en poids, rapporté au poids total de l'interface micro-adhérente, d'au moins un composé choisi parmi les oligosaccharides polysulfatés de 1 à 4 oses, leurs sels et leurs complexes.

Selon un mode de mise en oeuvre préféré, le pansement présente une libération prolongée dudit composé, caractérisée par une dissolution supérieure ou égale à 4 % en 5h, supérieure ou égale à 5 % en 10h, supérieure ou égale à 5,5 % en 15h, supérieure ou égale à 6 % en 20h, telle que mesurée conformément à la méthode du sachet, dans un milieu de dissolution constitué de 40 mL de sérum physiologique.

Selon un mode de mise en oeuvre préféré, le pansement présente une libération prolongée dudit oligosaccharide, caractérisée par une dissolution supérieure ou égale à 5 % en 5h, supérieure ou égale à 5,5 % en 10h, supérieure ou égale à 6 % en 15h, supérieure ou égale à 7 % en 20h, telle que mesurée conformément à la méthode du sachet, dans un milieu de dissolution constitué de 40 mL de sérum physiologique.

Selon un mode de mise en oeuvre préféré de l'invention, ladite interface micro-adhérente confère au pansement un pouvoir adhésif sur plaque d'acier compris entre 0,5 et 100 cN/cm, et de préférence compris entre 5 et 40 cN/cm.

Selon un autre mode de réalisation, l'invention consiste en un pansement, pour son utilisation dans la détersion ou la cicatrisation d'une plaie, pour son utilisation dans la prévention et le traitement des vergetures ainsi que dans la prévention et le traitement des cicatrices, notamment des cicatrices pathologiques.

Selon un dernier mode de réalisation, l'invention consiste en un procédé de fabrication d'un pansement comprenant :

(a) le dépôt ou l'incorporation, respectivement sur ou dans l'interface micro-adhérente du pansement, d'au moins un composé choisi parmi les oligosaccharides polysulfatés comprenant 1 à 4 unités oses, leurs sels et leurs complexes,

(b) un traitement par de l'oxyde d'éthylène dudit pansement.

Selon un mode de mise en oeuvre préféré l'étape (b) de traitement par de l'oxyde d'éthylène comporte au moins les étapes suivantes :

(i) une étape de pré-conditionnement,
(ii) une étape d'exposition à l'oxyde d'éthylène,
(iii) une étape de rinçage.

Selon un mode de mise en oeuvre préféré :

- au cours de l'étape (i) de pré-conditionnement le pansement ou la couche active est soumis à une température allant de 25 à 60°C et un degré d'hygrométrie allant de 50 à 95% pendant une durée allant de 5 à 15h,
- l'étape (ii) comporte un traitement par de l'oxyde d'éthylène gazeux à une pression supérieure ou égale à 920 mBars dans une enceinte à un degré d'hygrométrie supérieur ou égal à 50, à une température supérieure ou égale à 30°C, et pendant une durée d'au moins 2 heures,
- l'étape (iii) comporte au moins deux séquences successives d'injection d'azote, suivie d'une détente.

Selon un dernier mode de réalisation l'invention consiste en un procédé de prévention et/ou de traitement cosmétique des vergetures, ou des cicatrices ou des lésions cutanées dues aux vergetures, ce procédé comprenant l'application sur la ou les zones de la peau concernées d'un pansement. Le même procédé étant applicable à la prévention et/ou au traitement cosmétique des cicatrices ou des lésions cutanées.

## Description détaillée

D'autres caractéristiques et avantages de l'invention apparaîtront plus clairement à la lecture de la description qui suit d'un mode de réalisation préféré de l'invention, donnée à titre d'exemple et en référence aux figures annexées.

De façon tout à fait surprenante, la Demanderesse a mis au point de nouveaux pansements comprenant une interface micro-adhérente à base d'oligosaccharides polysulfatés présentant une libération de principe actif prolongée et élevée.

Ces pansements sont obtenus par un procédé comprenant le dépôt ou l'incorporation, respectivement sur ou dans l'interface du pansement destinée à venir en contact avec la peau ou avec la plaie, d'au moins un composé choisi parmi les oligosaccharides polysulfatés comprenant 1 à 4 unités oses, ce procédé comprenant en outre un traitement par de l'oxyde d'éthylène.

Ces pansements comprennent au moins un composé choisi parmi les oligosaccharides polysulfatés comprenant 1 à 4 oses, et ils présentent un relargage prolongé et plus élevé en ces principes actifs, comparativement à des pansements n'ayant pas subi le traitement à l'oxyde d'éthylène.

Les pansements de l'invention comprennent et sont préparés à partir d'au moins un composé choisi parmi les oligosaccharides polysulfatés comprenant 1 à 4 unités d'oses, leurs sels et leurs complexes.

Les oligosaccharides susceptibles d'être utilisés dans la présente invention sont des oligomères formés de 1 à 4 unités monosaccharide, et de préférence de 1 ou 2 unités monosaccharide, généralement liées entre elles par liaison glycosidique alpha ou bêta. En d'autres termes, il s'agit de mono, di, tri ou tétrasaccharides, et de préférence de mono ou disaccharides. Il n'y a pas de limitation particulière concernant la nature des unités oses de ces polysaccharides. De préférence, il s'agit de pentoses ou d'hexoses.

A titre d'exemples de monosaccharides, on peut citer le glucose, le galactose ou le mannose. A titre d'exemples de disaccharides, on peut citer le maltose, le lactose, le sucrose ou le tréhalose. A titre d'exemple de trisaccharide, on peut citer le mélézitose. A titre d'exemple de tétrasaccharide, on peut citer le stachyose. De préférence, l'oligosaccharide est un disaccharide, de préférence encore le sucrose.

Par "oligosaccharide polysulfaté", on entend un oligosaccharide dont au moins deux, et de préférence tous les groupes hydroxyle de chaque monosaccharide ont été substitués par un groupe sulfate. De préférence, l'oligosaccharide polysulfaté est le sucrose octasulfate.

Les oligosaccharides polysulfatés utilisés dans le cadre de la présente invention peuvent se présenter sous forme de sels ou de complexes.

D'une manière générale, dans la présente demande, on inclut dans l'expression « oligosaccharides polysulfatés » les sels et les complexes de ces composés.

A titre d'exemple de sels, on peut citer les sels de métal alcalin tels que les sels de sodium, de calcium ou de potassium ; les sels de métal de transition tels que les sels d'argent, les sels de zinc, et les sels d'acide aminé.

A titre d'exemple de complexes, on peut citer les complexes avec l'hydroxyaluminium.

Des composés particulièrement préférés pour la mise en oeuvre de l'invention sont les suivants :

- le sel de potassium du sucrose octasulfate,
- le sel d'argent du sucrose octasulfate,
- le complexe d'hydroxyaluminium du sucrose octasulfate communément désigné par le nom sucralfate (pour sucrose aluminium sulfate).

De préférence le composé choisi parmi les oligosaccharides polysulfatés de 1 à 4 oses, leurs sels et leurs complexes est mis en oeuvre dans la fabrication du pansement sous forme micronisée.

Les composés décrits ci-dessus peuvent être utilisés seuls ou en mélange, ou encore en combinaison avec une (ou plusieurs) autre(s) substance(s) active(s) permettant d'induire ou d'accélérer la cicatrisation ou pouvant avoir un rôle favorable dans le traitement d'une plaie, ou pouvant traiter et/ou prévenir les vergetures ou les cicatrices, pathologiques ou non.

Parmi ces substances actives, on peut citer, en particulier, à titre d'exemples :

- les agents antibactériens tels que les sels ou complexes d'argent (tels les sulfates d'argent, les nitrates d'argent, les sulfamides d'argent ou encore les zéolites à base d'argent), les sels de zinc ou de cuivre, le métronidazole, la néomycine, les pénicillines, l'acide clavulanique, les tétracyclines, la mynocycline, la chlorotétracycline, les aminoglycosides, l'amikacine, la gentamicine, les probiotiques ;
- les antiseptiques tels que la chlorhexidine, le trichlosan, le biguanide, l'hexamidine, le thymol, le lugol, la povidone iodée, le chlorure de benzalkonium et de benzethonium ;
- les anti-douleurs tels que le paracétamol, la codéine, le dextropropoxyphène, le tramadol, la morphine et ses dérivés, les corticoïdes et leurs dérivés ;
- les anesthésiques locaux tels que la lidocaïne, la benzocaïne, la dibucaïne, le chlorhydrate de pramoxine, la bupivacaïne, la mépivacaïne, la prilocaïne, l'étidocaïne ;
- les anti-inflammatoires comme les anti-inflammatoires non stéroïdiens (AINS), l'aspirine ou acide acétylsalicylique, l'ibuprofène, le kétoprofène, le flurbiprofène, le diclofenac, l'acéclophénac, le kétorolac, le méloxicam, le piroxicam, le ténoxicam, le naproxène, l'indométacine, le naproxcinod, le nimésulid, le célécoxib, l'étoricoxib, le parécoxib, le rofécoxib, le valdécoxib, la phénylbutazone, l'acide niflumique, l'acide méfénamique;

Bien entendu, les composés utilisés dans les pansements selon l'invention peuvent aussi être utilisés en association avec un ou plusieurs autres composés connus pour leur action dans la phase de détersion comme par exemple :

- des enzymes ;
- l'urée.

Les composés oligosaccharides polysulfatés utilisés dans les pansements selon l'invention peuvent aussi être utilisés en association avec un ou plusieurs autres composés connus pour le traitement des vergetures, tels que :

- les dérivés de l'acide rétinoïque
- les acides de fruits.

Les composés oligosaccharides polysulfatés utilisés dans les pansements selon l'invention peuvent aussi être utilisés en association avec un ou plusieurs autres composés connus pour leur action cicatrisante, tels que :
Le rétinol, la vitamine A, la vitamine E, la N-acétyl-hydroxyproline, les extraits de *centella asiatica*, la papaïne, les huiles essentielles de thym, de niaouli de romarin, de sauge, l'acide hyaluronique, l'allantoïne, l'urée, les enzymes protéolytiques tels que la streptokinase, la tripsine ou la collagénase, des inhibiteurs de protéases.

Dans le cadre de la présente invention les composés oligosaccharides polysulfatés sont intégrés à un pansement par l'intermédiaire d'une masse d'enduction ou d'imprégnation constitutive de l'interface micro-adhérente.

Par pansement, on entend dans la présente invention tout dispositif médical de type pansement comprenant au moins une interface micro-adhérente.

Notamment l'invention s'applique aux pansements utilisés pour le traitement des plaies, et à ceux utilisés pour le traitement des cicatrices, aux patchs cosmétiques.

Le choix du pansement dépend du type de lésion à traiter. Par exemple, les pansements absorbants sont favorisés dans le cas du traitement de plaies car durant la phase de détersion celles-ci sont souvent très exsudatives.

De préférence, afin de favoriser une action rapide, ce composé oligosaccharidique polysulfaté (ou la masse d'enduction ou d'imprégnation le comprenant) est incorporé dans la couche du pansement qui vient en contact avec la peau ou déposé sur la surface du pansement qui vient en contact avec la peau.

De telles techniques de dépôt sont bien connues de l'homme de l'art et certaines sont par exemple décrites dans la demande de brevet WO 2006/007844.

Avantageusement, si le composé oligosaccharide polysulfaté n'est pas contenu dans une masse d'enduction ou d'imprégnation, celui-ci est déposé sur la surface de la couche destinée à venir au contact de la peau, couche qui aura subi une étape d'enduction ou d'imprégnation préalable à l'aide de la masse d'enduction ou d'imprégnation dépourvue du composé oligosaccharide polysulfaté :

- soit sous forme liquide, par exemple par vaporisation d'une solution ou suspension le comprenant ;
- soit sous forme solide, par exemple par tamisage et/ou projection d'une poudre le comprenant.

En revanche, dans la configuration où le composé oligosaccharide polysulfaté est compris au préalable dans une masse d'enduction ou d'imprégnation, celle-ci est déposée sur la surface de la couche destinée à venir au contact de la peau soit de façon continue (par enduction unie, par enduction pleine ou par enduction d'une trame) soit de façon discontinue (par stries d'enduction parallèles).

On peut également prévoir de formuler directement le composé oligosaccharide polysulfaté dans la composition d'une couche particulière du pansement au cours de la fabrication de celle-ci.

La quantité de composé oligosaccharide polysulfaté utilisée dans le pansement est adaptée en fonction de la cinétique de relargage recherchée. Avantageusement on prévoit que le pansement comprenne de 0,5 à 2 mg/cm2, préférentiellement de 0,7 à 1,9 mg/cm2, avantageusement de 0,9 à 1,7mg/cm2 de principe actif oligosaccharide polysulfaté. Cette valeur est rapportée à la surface active du pansement, c'est-à-dire la surface destinée à relarguer du principe actif, les parties du pansement non actives, également appelées « trottoir », étant généralement celles permettant la fixation de celui-ci autour de la zone à traiter.

Dans le cadre de son utilisation dans un élément de pansement, le composé oligosaccharide polysulfaté est incorporé en une quantité telle que la quantité de ce composé relarguée est comprise entre 70 et 140 $\mu$g/cm2, et de préférence entre 80 et 130 $\mu$g/cm2 au bout de 24 heures. Cette valeur est rapportée à la surface active du pansement.

Les pansements selon la présente invention comprennent au moins une structure interface micro-adhérente telle qu'une couche élastomérique sur laquelle est déposé, ou à laquelle est incorporé, l'oligosaccharide polysulfaté.

Parmi les pansements connus qui peuvent être utilisés dans le cadre de la présente invention, on peut mentionner par exemple:
Les films de polyuréthane, tels que par exemple les produits commercialisés par la société Smith&Nephew sous la marque Opsite®, ou par la société 3M sous la marque Tegaderm® ou encore par les Laboratoires URGO sous la marque Optiskin®. Ces pansements sont constitués d'un film mince (de l'ordre de 20 à 50 $\mu$m) transparent de polyuréthane adhésivé. Leur transparence permet un contrôle visuel de la zone à traiter. Ces films de polyuréthane sont semi-perméables, ils sont perméables aux échanges gazeux, et sont imperméables aux liquides et aux bactéries. Ils confèrent une protection mécanique vis-à-vis des phénomènes de frottement, friction et cisaillement.

Les pansements hydrocellulaires, tels que par exemple les produits commercialisés par Môlnlycke sous la marque Mepilex® ou par Smith & Nephew sous la marque Allevyn®, ou encore par les Laboratoires URGO sous la marque Cellosorb®. Ces pansements sont généralement constitués d'un support qui peut être un film de polyuréthane ou un non tissé, d'une couche absorbante qui peut être une mousse de polyuréthane. La face destinée à venir en contact avec la plaie de cette couche absorbante peut être couverte d'une masse d'enduction micro-adhérente ou non. Ces pansements possèdent une capacité d'absorption élevée, par capillarité et/ou par rétention au sein de la structure hydrocellulaire.

Les pansements hydrofibres, tels que par exemple les produits commercialisés par Convatec sous la marque Aquacel®. Ces pansements sont des fibres non tissées d'hydrocolloïdes purs (carboxyméthylcellulose sodique). Ces pansements sont très hydrophiles et se transforment en un gel cohésif au contact des exsudats. Ils possèdent une capacité d'absorption très élevée et permettent également de « piéger » les bactéries, contrôlant ainsi la contamination bactérienne.

Les alginates, tels que par exemple les produits commercialisés par Smith & Nephew sous la marque Algisite® ou par Coloplast sous la marque Seasorb® soft ou encore par les Laboratoires URGO sous la marque Urgosorb®. Ces pansements se présentent généralement sous la forme de compresses ou de mèches. Ils sont constitués de polysaccharides naturels et gélifient au contact des exsudats. Ils possèdent une capacité d'absorption très élevée et peuvent également « piéger » les bactéries, contrôlant ainsi la contamination bactérienne.

Les pansements hydrocolloïdes, tels que par exemple les produits commercialisés par Convatec sous la marque Duoderm® ou par Coloplast sous la marque Comfeel® ou encore par les Laboratoires URGO sous la marque Algoplaque® ou, dans une application grand public, par Johnson & Johnson sous la marque Compeed® ou encore par les Laboratoires URGO sous la marque URGO® Ampoules. Ces produits sont généralement constitués d'un support qui est un film de polyuréthane et d'une masse élastomérique adhésive contenant des hydrocolloïdes. Ces pansements sont hydrophiles et la masse élastomérique adhésive qui contient les hydrocolloïdes se gélifie au contact des exsudats. Ils adhèrent à la peau saine, mais pas à la plaie.

Les pansements selon la présente invention, tels que ceux décrits précédemment, comprennent au moins une structure interface micro-adhérente telle qu'une couche élastomérique sur laquelle est déposé, ou à laquelle est incorporé, l'oligosaccharide polysulfaté. Cette interface micro-adhérente est destinée à venir en contact avec la peau ou avec la plaie.

Afin de ne pas altérer les tissus sains ou les berges de la plaie, notamment lors du retrait du pansement, on préfère un adhésif ayant la propriété d'adhérer à la peau sans adhérer à la plaie. A titre d'exemple d'un tel adhésif, on peut ainsi citer les adhésifs à base d'élastomères de silicone ou de polyuréthane, tels que les gels de silicone ou de polyuréthane et les adhésifs hydrocolloïdes.

De préférence les pansements de l'invention comprennent une interface micro-adhérente choisie parmi les couches de masse ou de composition adhésive élastomérique, dans laquelle sont incorporés le ou les composés oligosaccharide polysulfaté.

De telles compositions adhésives élastomériques sont constituées d'une matrice élastomérique à base d'un ou de plusieurs élastomères choisis parmi les polymères séquencés poly(styrène-oléfine-styrène) en association avec un ou plusieurs composés choisis parmi les huiles plastifiantes, telles que les huiles minérales, mais aussi associé à des résines tackifiantes, ou encore à une quantité, de préférence faible d'hydrocolloïde (de 3 à 20% en poids) comme par exemple la carboxyméthylcellulose de sodium et, si nécessaire, des antioxydants.

Les formulations de telles masses adhésives élastomériques sont bien connues et décrites par exemple dans la demande de brevet FR 2 916 356.

Le composé oligosaccharide polysulfaté est utilisé de préférence en une quantité comprise entre 1 et 15% en poids, et de préférence encore entre 5 et 10% en poids, rapportée au poids total de la composition adhésive.

De façon avantageuse, dans le pansement selon l'invention la couche de masse adhésive élastomérique comprend notamment les éléments suivants : un composé élastomérique, un hydrocolloïde, au moins une résine tackifiante et au moins une huile plastifiante. En outre, elle comprend au moins un composé choisi parmi les oligosaccharides polysulfatés de 1 à 4 oses, leurs sels et leurs complexes.

La couche de masse adhésive élastomérique permet de former une couche d'interface micro-adhérente constituée par une matrice lipido-colloïde permettant de faciliter la pose mais aussi le retrait et le positionnement atraumatique du pansement. Cette fixation provisoire peut également aider le personnel soignant ou l'utilisateur à fixer le pansement à l'aide d'autres moyens de fixation, e.g. à recouvrir le pansement d'un moyen de contention ou d'un ruban adhésif. Dans ce cas, la couche d'interface peut être choisie de telle sorte que le pansement présente un pouvoir adhésif sur plaque d'acier compris entre 0,5 et 100 cN/cm, de préférence entre 5 et 40 cN/cm. Ce pouvoir adhésif est mesuré selon la méthode EN 1939 dans laquelle un échantillon de pansement de 20 mm de large et 150 mm de long est posé sur une plaque d'acier et dans laquelle on mesure, au bout de 10 minutes, le pouvoir adhésif avec un dynamomètre à une vitesse de traction de 100 mm/min avec un angle de 90°. Par composé élastomérique, on entend tous les polymères séquencés poly(styrène-oléfine-styrène) triblocs, éventuellement associés à des copolymères diblocs. Les copolymères triblocs peuvent être des copolymères séquencés poly(styrène-éthylène-butylène-styrène), appelés aussi SEBS, vendus sous la dénomination Kraton G1651®, Kraton G1654® ou encore Kraton G1652®. Les copolymères diblocs peuvent être des copolymères séquencés poly(styrène-éthylène-propylène-styrène), appelés aussi SEPS. Le composé élastomérique peut également être choisis parmi les élastomères appartenant à la famille des copolymères triblocs poly(styrène-isoprène-styrène) (en abrégé : poly(SIS)) et les mélanges de copolymères triblocs poly(SIS) et de copolymères diblocs poly(styrène-isoprène).

Par hydrocolloïdes, on entend tout composé hydrocolloïdique approprié, comme par exemple, la pectine, les alginates, les gommes végétales naturelles (gomme de Karaya), les dérivés de cellulose tels que les carboxyméthylcelluloses et leurs sels de métal alcalin (les sels de sodium ou de calcium de carboxyméthylcellulose connus sous la référence de CMC Blanose 7H4XF®), ainsi que les polymères synthétiques à base de sels de l'acide acrylique superabsorbant, comme entres autres les produits commercialisés par la société BASF® sous la dénomination Luquasorb 1003®, ou par la société CIBA Speciality Chemicals® sous la dénomination Salcare SC91®, ainsi que les mélanges de ces composés. Ces hydrocolloïdes sont avantageusement mis en oeuvre sous forme de particules pour la préparation de la composition adhésive.

Les résines tackifiantes entrant dans la composition des masses adhésives hydrocolloïdes sont choisies notamment parmi les polyisobutylènes à bas poids moléculaire. De façon générale, on préfère l'utilisation de résines hydrogénées telles que les résines Escorez® de la série 5000, et encore plus préférentiellement, la résine Escorez 5380®.

Parmi les huiles plastifiantes utilisables pour la mise en oeuvre de l'invention, on peut citer notamment les huiles minérales, les polybutènes ou encore les dérivés de phtalate. De manière préférentielle, on utilise une huile plastifiante minérale choisie parmi les produits commercialisés par la société Shell® sous la dénomination Ondina 917®, Ondina 919® ou encore Ondina 933®. Selon un mode de réalisation particulier, une quantité d'huile Ondina® choisie peut être substituée par une quantité équivalente en vaseline Codex A® commercialisée par la société Aiglon®.

La couche de masse adhésive hydrocolloïde peut comprendre des composés supplémentaires tels qu'un antioxydant, un agent stabilisant et un composé plastifiant.

Par « antioxydant », on entend toute molécule qui diminue ou empêche l'oxydation d'autres substances chimiques. L'antioxydant pourra être choisi parmi les antioxydants phénoliques, comme par exemple les produits commercialisés par la société CIBA-GEIGY® sous la dénomination Irganox 1010®, Irganox 565® et Irganox 1076® ainsi que les antioxydants souffrés, comme par exemple le dibutyldithiocarbamate de zinc commercialisé par la société AKZO® sous la dénomination PERKACIT ZDBC®. Préférentiellement, l'antioxydant utilisé sera l'Irganox 1010®.

Par agent stabilisant, on entend tout composé permettant d'optimiser la vitesse de gélification, la mouillabilité voire même le relargage d'actifs éventuellement présents dans la composition, tel que le polymère SEPINOV® EMT 10 commercialisé par la société SEPPIC, également connu sous la dénomination de copolymère de sel de l'acide 2-méthyl-2[(1-oxo-2-propényl)amino]-1-propanesulfonique et de l'ester 2-hydroxyéthyle de l'acide propénoïque.

De préférence, outre le ou les oligosaccharides polysulfatés, la couche de masse adhésive élastomérique consiste essentiellement en :

10 à 60 % en poids d'au moins une résine tackifiante,
2 à 20% en poids, de préférence 12 à 16 % en poids d'au moins un composé hydrocolloïde,
10 à 65 % en poids d'au moins une huile minérale plastifiante,
3 à 25 % en poids d'au moins un polymère élastomérique.

De préférence, si le composé oligosaccharide polysulfaté est compris dans la couche de masse adhésive élastomérique, cette dernière consiste essentiellement en :

10 à 60 % en poids d'au moins une résine tackifiante,
2 à 20% en poids, de préférence 12 à 16 % en poids d'au moins un composé hydrocolloïde,
10 à 65 % en poids d'au moins une huile minérale plastifiante,
3 à 25 % en poids d'au moins un polymère élastomérique,
1 à 15 % en poids d'oligosaccharide polysulfaté.

De façon préférée, outre le ou les oligosaccharides polysulfatés, la couche de masse adhésive élastomérique consiste essentiellement en :

0,05 à 1% en poids d'au moins un agent antioxydant,
10 à 60 % en poids d'au moins une résine tackifiante,
2 à 20% en poids, de préférence 12 à 16 % en poids d'au moins un composé hydrocolloïde,
10 à 65 % en poids d'au moins une huile minérale plastifiante,
3 à 25 % en poids d'au moins un polymère élastomérique,
1 à 15 % en poids d'au moins un agent stabilisant.

De façon préférée, si le composé oligosaccharide polysulfaté est compris dans la couche de masse adhésive élastomérique, cette dernière consiste essentiellement en :

0,05 à 1% en poids d'au moins un agent antioxydant,
10 à 60 % en poids d'au moins une résine tackifiante,
2 à 20% en poids, de préférence 12 à 16 % en poids d'au moins un composé hydrocolloïde,
10 à 65 % en poids d'au moins une huile minérale plastifiante,
3 à 25 % en poids d'au moins un polymère élastomérique,
1 à 15 % en poids d'au moins un agent stabilisant,
1 à 15 % en poids d'oligosaccharide polysulfaté.

Dans le cadre de la présente invention, la couche d'interface micro-adhérente, également appelée parfois masse micro-adhérente peut éventuellement être associée à au moins un des composés choisis parmi le groupe constitué d'une couche support, d'une couche absorbante, et d'un voile non absorbant. De préférence, la couche d'interface micro-adhérente est associée soit à une couche absorbante seule, soit à une couche support, un voile non absorbant et une couche absorbante, soit à une couche support seul, ou encore soit à une couche support et une couche absorbante.

Par « couche support », on entend une couche qui, dans le cadre de la présente invention, peut être constitué de matériaux polymériques de nature variée tels qu'un polyamide, polyuréthane, polyester, polyéther, polychlorure de vinyle, polychlorure de vinylidène, alcool polyvinylique, polyacétate de vinyle, polystyrène, fluorure polyvinylique, une polyoléfine comme par exemple un polyéthylène ou un polypropylène, un matériau à base de copolymère de polyéther polyester, de copolymère de polyester ou polyéther polyuréthane, de copolymère polyéther polyamide.

La couche support peut être formée d'un tissu de fils en matériau flexible et très peu extensible et non élastique. Ce support se présente sous forme d'un tissu à mailles larges ouvertes et peut être obtenu par des procédés de tissage ou de tricotage permettant de former des mailles ouvertes de taille régulière, carrées ou polygonales. Dans le cas d'un tissage, les mailles peuvent être fixées au moyen de fils de tour afin d'obtenir une bonne stabilité dimensionnelle. La dimension des mailles est telle que la surface unitaire des ouvertures est de l'ordre de 0,2 à 10 mm$^2$, préférentiellement 0,5 à 10 mm$^2$ et plus préférentiellement 0,5 à 3 mm$^2$, le taux d'ouverture du tissu (rapport de la surface ouverte sur la surface totale) étant de l'ordre de 50 à 90 %. Le fil utilisé pour fabriquer le tissu est préférentiellement un fil continu à filaments, très peu extensible et non élastique, l'extensibilité ou l'allongement à la rupture étant inférieure à 35 %. Par fil continu à filaments, on entend un fil formé de un ou plusieurs filaments longs retords ; le choix de filaments longs permet d'éviter les fibres courtes qui risquent de se détacher du support et venir se disperser près de la surface de contact avec la plaie. Pour la même raison, le matériau constitutif des fils est de préférence de type hydrophobe, de nature artificielle ou synthétique ; ces constituants, comme par exemple les polyesters, les polyamides, les acétates de cellulose permettent d'obtenir des filaments longs et des fils présentant beaucoup moins de fibrilles que les fils obtenus à partir de fibres courtes par exemple. Le choix de certains matériaux synthétiques tels que des polyesters donne également la possibilité de thermofixer la structure à mailles larges du support. Le tissu à mailles larges est préférentiellement réalisé avec des fils de même nature, mais on peut utiliser aussi des tissus fabriqués par exemple avec des fils de chaîne et des fils de trame qui seraient de nature différente. La nature du fil est, par exemple, un polyester de type polyéthyltéréphtalate, un polyamide ou un acétate de cellulose ; on utilise de préférence un tissu à mailles larges thermofixées en fils continus de polyester (Tergal ou polyéthyltéréphtalate), par exemple des tissus commercialisés sous le nom de marquisette, grammant environ 20 à 80 g/m2 et préférentiellement 30 à 80 g/ m2. Ces tissus pratiquement non extensibles dans les directions chaîne ou trame présentent l'avantage de se travailler plus facilement que les tissus élastiques et on obtient un enrobage plus régulier des fils.

La couche support peut être adhésivée ou non sur la face destinée à venir en contact avec la peau ou la plaie.

La couche support peut être monocouche ou multicouche, comme par exemple un film bicouche dont la seconde couche forme la surface opposée à celle destinée à venir en contact avec la peau ou la plaie. Cette seconde couche peut être constituée d'un matériau polymérique de même nature que ceux mentionnés précédemment ou bien encore de papiers siliconés ou non.

Préférentiellement, la couche support peut être constituée d'un film continu et d'une armature ajourée enduite d'un gel de silicone adhésif du type polydiméthylsiloxane, commercialisée sous la référence Novésil® par la société Zodiac®.

Dans ce cas, la couche peut être constituée d'un film ou d'un complexe quelconque intégrant un film. Parmi les films utilisables, on peut citer à titre d'exemple les films en polyuréthane, en polyétheruréthane, en polyétheramide, ou en polyétherester.

L'armature quant à elle, peut être constituée de tout matériau ajouré tels un film perforé, un filet thermoplastique, un tissé, un tricot, ou un non tissé, de préférence élastique pour une meilleure tenue du pansement sur la peau. Un film perforé peut être par exemple en polyéthylène ou en polypropylène. Un textile tissé peut être par exemple en polyéthylène téréphtalate ou en polyamide.

Par couche absorbante, on entend au sens de la présente invention tout matériau ou association de matériaux utilisés pour la réalisation d'une couche absorbante dans le domaine des pansements ou des produits d'hygiène, telles les couches culotte.

Parmi ces matériaux, on peut citer les mousses absorbantes hydrophiles, par exemple à base de polyu

réthane, des matériaux textiles notamment des tissés et des non tissés à base de fibres absorbantes ou de fibres gélifiantes, des matériaux super-absorbants par exemple à base de polymères acryliques notamment sous forme de particules ou de fibres, des compositions de préférence adhésives contenant des particules d'hydrocolloïdes et des hydrogels.

A titre d'exemple de couche absorbante, on peut citer les mousses commercialisées par les sociétés CORPURA et RYNEL respectivement sous les références MCF03 et L 00562-B.

Une catégorie de non tissé est représentée par les non tissés à base de fibres de cellulose.

Ces non tissés peuvent aussi incorporer des particules de polymères superabsorbants couramment désignées sous le terme de SAP tels des polymères acryliques (polyacrylates de sodium) dans une proportion comprise entre 10 et 60% en poids du poids total de la compresse afin d'augmenter leur capacité d'absorption. De même, afin de favoriser l'intégrité du non tissé lors de l'absorption, les fibres absorbantes peuvent être associées à des fibres non absorbantes telles des fibres thermo-liantes ou liées entre elles à l'aide de latex tel un latex EVA. Tous ces non tissés absorbants sont bien connus de l'homme du métier et désignés sous les termes de "hybrid bonded" ou "multibonded" airlaid (voir par exemple WO95/30394 ou WO94/10954).

Accessoirement, on peut utiliser comme couche absorbante une combinaison des différents non tissés cités précédemment.

L'utilisation de non tissés à base de fibres gélifiantes est aussi bien connue de l'homme de l'art. On peut citer, à titre d'exemple, comme fibres gélifiantes les fibres à base d'acide hyaluronique, de chitosan, de pectine, d'alginates, de carboxyméthylcellulose de sodium, de carboxyméthylcellulose de sodium associée aux alginates, de fibres de cellulose chimiquement modifiées, en particulier carboxyméthylées, ou les fibres à base de polymères super-absorbants.

A titre d'exemple, on peut citer les fibres commercialisées sous les dénominations Lanseal F.

Comme précédemment ces fibres gélifiantes peuvent être associées à d'autres types de fibres pour améliorer les propriétés du non tissé comme par exemple des fibres thermo-liantes.

De tels non tissés et les différentes fibres qui peuvent les composer sont décrits par exemple dans les demandes de brevet suivantes : WO 2007/025546, WO 2007/08531, WO 93/12275, WO 00/01425, WO 94/16746, WO 95/19795, EP 878 204, EP 1 435 247 ou WO 86/01400.

Par super-absorbant on entend ici des polymères sous formes de poudres, de fibres ou tout autre forme qui au contact des liquides biologiques gélifient.

Des polymères hydrophiles sous forme de particules présentent des propriétés super-absorbantes sont décrits par exemple dans la demande US 4, 102, 340. En particulier des matériaux absorbants comme les polyacrylamides réticulés sont utilisés pour cela. Des particules super-absorbantes préférées sont composées d'acide polyacrylique réticulé partiellement neutralisé. On peut citer à titre d'exemples les produits commercialisés par la société BASF sous la dénomination LUQUASORB ou ceux commercialisés par la société Ciba Speciality Chemicals sous la dénomination SALCARE.

Ces super-absorbants sont utilisés de façon générale en association avec des fibres de cellulose comme décrits précédemment ou incorporés dans des compositions de préférence adhésives utilisées dans les pansements hydrocolloïdes.

La couche absorbante peut aussi se composer de ces super-absorbants, seuls ou incorporés entre 2 couches de distribution, ou à un non tissé de fibres absorbantes comme par exemple des fibres de cellulose ou de viscose (voir EP 358412 ou US 6 096 942).

Par voile non absorbant, on entend un voile intercalé entre la mousse absorbante et le support, et destiné à les assembler. Ce moyen de fixation est nécessaire car la surface du support enduite d'un gel de silicone adhésif n'adhère pas de façon suffisante à la mousse absorbante, en particulier en milieu humide.

Le voile intercalé entre la mousse absorbante et le support est non tissé, non absorbant, et de faible grammage. Le non tissé peut être choisi parmi tout type de non tissé couramment utilisé dans le domaine de l'hygiène et des pansements, notamment un non tissé filé lié (Spun laid), cardé (Carded) ou aiguilleté (Spun lace).

Il peut être constitué de fibres de polyamide, de polyester, de polyuréthane et/ou de polyoléfines. Selon un mode de réalisation le voile comprend des fibres de polyéthylène. Les fibres peuvent être monocomposants, ou bicomposants de type coeur/écorce ou côte-à-côte. On choisira par exemple un non tissé spun laid, de préférence de type spunbond.

Le voile non absorbant est de préférence constitué de fibres hydrophobes, mais il peut aussi être constitué de fibres hydrophiles et avoir subi un traitement pour le rendre hydrophobe. Le voile peut être constitué de plusieurs couches, dans la mesure où sa porosité est suffisante, la couche venant au contact du gel de silicone adhésif étant non absorbante et de préférence hydrophobe.

Le voile est fixé à la mousse absorbante sur toute sa surface, ou de préférence seulement sur sa périphérie par les technologies classiques de fixation telles que la chaleur, les ultrasons, la haute fréquence, ou par des adhésifs.

Selon l'invention, le pansement comprenant au moins un composé choisi parmi les oligosaccharides polysulfatés comprenant 1 à 4 unités oses, leurs sels et leurs complexes est fabriqué par un procédé comprenant au moins un traitement à l'oxyde d'éthylène. Ce traitement à l'oxyde d'éthylène est avantageusement appliqué au pansement complet.

De préférence, ce procédé comporte au moins 3 étapes :

(i) une étape de pré-conditionnement,
(ii) une étape d'exposition à l'oxyde d'éthylène,
(iii) une étape de rinçage.

L'étape (i) de pré-conditionnement consiste à soumettre le pansement à une température allant de 25 à 60°C et un degré d'hygrométrie allant de 50 à 95%.

De préférence durant cette étape la température est de 30 à 55°C, avantageusement de 35 à 50°C, de préférence de 40 à 45°C. De préférence durant cette étape le degré d'hygrométrie est de 55 à 90%, préférentiellement de 60 à 80%, préférentiellement de 65 à 75%. La durée du traitement est avantageusement comprise entre 5 et 15h, de préférence de 9 à 12h. De façon connue, l'étape de conditionnement peut être mise en oeuvre dans une chambre climatique conditionnée, comme par exemple dans une étuve.

Les pansements sont ensuite transférés dans une autre chambre, laquelle est soumise à une injection d'azote gazeux puis à une injection d'oxyde d'éthylène ou bien à une injection simultanée de ces deux gaz, pour obtenir une pression finale comprise entre 920 et 960 mBars ; la pression partielle d'oxyde d'éthylène étant quant à elle comprise entre 300 et 550 mBars, cette dernière pouvant être contrôlée par la masse d'oxyde d'éthylène introduite dans l'étuve.

De préférence, le degré d'hygrométrie dans la chambre de traitement de l'étape (ii) est supérieur ou égal à 50%.

Avantageusement le % relatif [N2]/[OE] (en mole) de N2 et d'oxyde d'éthylène (OE) injecté dans la chambre vérifie :

$$0,85 \leq [N2]/[OE] \leq 2,15$$

La masse d'oxyde d'éthylène peut être contrôlée de façon indirecte, c'est-à-dire à l'aide d'un débitmètre massique ou volumique ou par simple pesée.

Avantageusement, l'étape (ii) comporte une élévation de la température à un niveau supérieur ou égal à 30°C, de préférence supérieur ou égal à 35°C, avantageusement supérieur ou égal à 40°C pendant une durée d'au moins 2 heures, de préférence au moins 4 heures, avantageusement au moins 6 heures. Cette élévation de température est appliquée de façon simultanée au maintien sous pression d'oxyde d'éthylène du pansement. La dernière étape (iii) consiste alors à effectuer un ou plusieurs rinçages, par injection d'azote dans la chambre, suivie d'une détente. De préférence on effectue au moins deux rinçages successifs. De préférence l'injection d'azote est effectuée à une pression supérieure ou égale à 920 mBars.

Il s'agit, selon un mode de réalisation préféré de l'invention, d'un pansement comportant au moins une couche à base d'au moins un composé oligosaccharide polysulfaté possédant 1 à 4 oses, traité à l'oxyde d'éthylène, présentant une libération prolongée dudit oligosaccharide, caractérisée par une dissolution supérieure ou égale à 4 % en 5h, supérieure ou égale à 5 % en 10h, supérieure ou égale à 5,5 % en 15h, supérieure ou égale à 6 % en 20h, telle que mesurée conformément à la méthode du sachet, dans un milieu de dissolution constitué de 40 mL de sérum physiologique.

Avantageusement, le pansement de l'invention présente une libération prolongée dudit oligosaccharide, caractérisée par une dissolution supérieure ou égale à 5 % en 5h, supérieure ou égale à 5,5 % en 10h, supérieure ou égale à 6 % en 15h, supérieure ou égale à 7 % en 20h, telle que mesurée conformément à la méthode du sachet, dans un milieu de dissolution constitué de 40 mL de sérum physiologique.

De façon avantageuse, ladite interface micro-adhérente confère au pansement un pouvoir adhésif sur plaque d'acier compris entre 0,5 et 100 cN/cm, et de préférence compris entre 5 et 40 cN/cm. Ce pouvoir adhésif est mesuré selon la méthode EN 1939.

La méthode du sachet comporte les étapes suivantes : découpage d'échantillons de pansement et incorporation dans un sachet perméable, introduction de ceux-ci dans un récipient et mise en contact avec du sérum physiologique ; récupération du liquide au bout d'une durée déterminée ; mesure de la quantité de principe actif dans le liquide.

L'invention concerne encore un pansement tel que décrit ci-dessus, pour son utilisation en tant que dispositif médical. Selon un mode de réalisation, le pansement de l'invention est utilisé dans la détersion d'une plaie. Selon un mode de réalisation, le pansement de l'invention est utilisé pour favoriser la cicatrisation de la plaie. La présente invention concerne une méthode de traitement des plaies qui comprend l'utilisation d'un pansement tel que décrit ci-dessus. Dans le cadre de cette méthode de traitement, ce pansement est appliqué sur la plaie. L'invention concerne en particulier la détersion et la cicatrisation des brûlures et plaies aiguës ou chroniques. Elle concerne notamment la détersion et/ou la cicatrisation des brûlures, des radiodermites, des irritations d'origines diverses, des dermites, des écorchures, des éraflures, des égratignures, des coupures, des ulcères de jambes, des escarres, des plaies dues à un diabète, de l'acné cicatriciel, des ampoules, des chéilites, de l'eczéma, des érythèmes fessiers, des dermatoporoses.

L'activité d'agent de détersion des composés utilisés dans les pansements selon l'invention a été mise en évidence notamment dans le document FR 2 956 322. L'activité d'agent cicatrisant et/ou d'agent anti-inflammatoire des composés utilisés dans les pansements selon l'invention a été mise en évidence notamment dans le document FR 2 824 474.

La méthode de traitement des plaies selon l'invention est particulièrement avantageuse dans le cadre de la détersion autolytique, où le pansement absorbant, couramment utilisé dans cette technique, permet d'obtenir un produit optimal qui combine absorption des débris et action de dégradation du tissu fibrineux.

Selon un autre mode de réalisation, le pansement de l'invention est utilisé dans la prévention et le traitement des vergetures. En effet, les composés de type oligosaccharide polysulfaté ont montré une efficacité significative dans la prévention et le traitement des vergetures comme décrit dans la demande déposée sous le numéro FR 11 56431. Les pansements de l'invention peuvent être utilisés avant ou durant ou après la phase de formation des vergetures pour prévenir, éviter, retarder et/ou diminuer l'apparition sur la peau de cicatrices ou lésions dues aux vergetures. Ces cicatrices ou lésions ne présentent pas un caractère pathologique mais ont un aspect disgracieux. En particulier les pansements de l'invention peuvent être utilisés de façon préventive chez des individus présentant un risque élevé de développer des vergetures (grossesse notamment). Ils peuvent également être utilisés de façon postérieure à la formation des vergetures pour réduire, diminuer, atténuer et/ou faire disparaitre les cicatrices ou lésions cutanées dues aux vergetures. L'invention a encore pour objet un procédé de prévention et/ou de traitement cosmétique des vergetures, ou des cicatrices ou des lésions cutanées dues aux vergetures, ce procédé comprenant l'application sur la ou les zones de la peau concernées d'un pansement selon l'invention. Selon l'invention, cette application peut avoir lieu avant, pendant ou après la formation des vergetures.

Selon un autre mode de réalisation, le pansement de l'invention est utilisé dans la prévention et le traitement des cicatrices, notamment des cicatrices pathologiques, telles que par exemple les cicatrices d'acné, les cicatrices consécutives à une intervention chirurgicale, des cicatrices de cryothérapie, des cicatrices post acte de dermatologie esthétique, en particulier les cicatrices hypertrophiques, rétractiles. En effet, les composés de type oligosaccharide polysulfaté ont montré une efficacité significative dans la prévention et le traitement des cicatrices comme décrit dans la demande déposée sous le numéro FR 11 56 436. Les pansements de l'invention peuvent être utilisés avant ou durant ou après la phase de formation des cicatrices pour prévenir, éviter, retarder et/ou diminuer l'apparition sur la peau des cicatrices. L'invention concerne notamment les cicatrices ou lésions ne présentant pas un caractère pathologique mais ayant un aspect disgracieux. En particulier les pansements de l'invention peuvent être utilisés de façon préventive chez des individus présentant un risque élevé de développer des cicatrices (acné notamment). Ils peuvent également être utilisés de façon postérieure à la formation des cicatrices pour réduire, diminuer, atténuer et/ou faire disparaitre les cicatrices ou lésions cutanées. L'invention a encore pour objet un procédé de prévention et/ou de traitement cosmétique des cicatrices ou des lésions cutanées, ce procédé comprenant l'application sur la ou les zones de la peau concernées d'un pansement selon l'invention. Selon l'invention, cette application peut avoir lieu avant, pendant ou après la formation des cicatrices ou des lésions cutanées.

La mise en oeuvre des composés oligosaccharide polysulfaté dans des pansements permettant leur relargage prolongé augmente l'efficacité de ces principes actifs par rapport aux différents modes de mise en oeuvre connus de l'art antérieur.

Enfin, un dernier mode de réalisation de l'invention consiste en un procédé de traitement à l'oxyde d'éthylène d'un pansement tel que ceux qui ont été décrits ci-dessus pour augmenter le relargage en oligosaccharides polysulfatés contenu dans ledit pansement.

**Figures**

- La figure 1 est une représentation graphique des profils de dissolution non cumulés et évalués en % de principe actif relargué à partir des pansements décrits à l'exemple 1 et à l'exemple 2.
- La figure 2 est une représentation graphique des profils de dissolution non cumulés et évalués en $\mu$g/mL de principe actif relargué à partir des pansements décrits à l'exemple 1 et à l'exemple 2.
- La figure 3 est une représentation graphique des profils de dissolution cumulés et évalués en % de principe actif relargué à partir des pansements décrits à l'exemple 1 et à l'exemple 2.
- La figure 4 est une représentation graphique des profils de dissolution cumulés et évalués en $\mu$g/mL de principe actif relargué à partir des pansements décrits à l'exemple 1 et à l'exemple 2.

Sur l'ensemble des figures, EI fait référence au pansement de l'exemple n°1 (selon l'invention) et E2 au pansement de l'exemple n°2 (comparatif).

**Partie expérimentale**

I- <u>Protocoles expérimentaux :</u>

<u>Protocole A : Fabrication et composition d'un pansement comprenant du sel de potassium de sucrose octasulfate dans une couche de composition hydrocolloïde</u>

Le pansement comprend une couche d'interface micro-adhérente à base d'hydrocolloïde, une mousse absorbante, un non tissé airlaid, un voile non absorbant de polyéthylène, et un support enduit de gel de silicone adhésif.

Les matériaux suivants sont utilisés :

- Le support est un tricot de polyester 40 g/m² enduit d'une masse silicone (200 g/m²), qui a été laminé sur un film de polyuréthane de 30 $\mu$m d'épaisseur présentant un taux de transmission de vapeur d'eau supérieur à 10 000 g/m²/24 heures. Ce support présente une épaisseur de l'ordre de 300 $\mu$m et une

MVTR de l'ordre de 5000 g/m$^2$/24 heures.

- Le voile non absorbant est un non tissé polyéthylène de 40 g/m$^2$ vendu sous la référence Vilmed® LSO 1040 WEISS par Freudenberg.
- La mousse absorbante est une mousse de polyuréthane hydrophile de 3 mm vendue par CORPURA sous la référence MCF 03.
- Un non tissé absorbant est inséré entre le voile et la mousse : c'est un non tissé airlaid (200 g/m$^2$) contenant un polymère super-absorbant de chez EAM Corporation vendu sous la référence Novathin®.
- Le protecteur pelable de 50 microns d'épaisseur en PET silico-fluoré est constitué de 2 parties ou ailettes se recouvrant sur la partie centrale de l'interface positionnée sur la plaie du patient; il est fourni par la société SILICONATURE qui le commercialise sous la référence SILFLU® M1R88001.

Le pansement est fabriqué selon le procédé suivant :

- Préparation de la couche d'interface et enduction sur la mousse absorbante :

On prépare la composition suivante, exprimée en pourcentage en poids par rapport au poids total:

- Huile minérale commercialisée par la société Shell sous la dénomination Ondina ®917 : 32,7%

- Sel de sodium de carboxyméthylcellulose commercialisé par la société AQUALON sous la dénomination CMC Blanose ® 7H4XF : 14%

- Copolymère séquencé de poly(styrène-éthylène-butylène) commercialisé par la société KRATON sous la dénomination KRATON® G 1654 : 6%

- Antioxydant commercialisé sous la dénomination IRGANOX® 1010 par la société CIBA SPECIALTY CHEMICALS : 0,1%

- Copolymère de sel de l'acide 2-méthyl-2[(1-oxo-2-propényl)amino]-1-propanesulfonique et de l'ester 2-hydroxyéthyle de l'acide propénoïque (agent de relargage) commercialisé par la société SEPPIC sous la dénomination SEPINOV® EMT 10 : 5%

- Résine tackifiante commercialisée par la société EXXON CHEMICALS sous la dénomination ESCOREZ® 5380 : 35%.

- Sel de potassium de sucrose octasulfate sous forme micronisé : 7.5%.

On a introduit l'huile minérale, l'hydrocolloïde, et l'élastomère, le KSOS puis l'antioxydant et l'agent de relargage et enfin la résine tackifiante portés à une température comprise entre 100 et 110°C dans un malaxeur MEL G-40, de manière à obtenir un mélange homogène.

Le mélange précédent est enduit de façon discontinue en une quantité de 170 g/m$^2$ ($\pm$ 40) sur la mousse polyuréthane hydrophile.

- Assemblage des couches:

- La feuille de voile de polyéthylène subit un traitement Corona dans les conditions suivantes.
- Puissance du générateur : 570 watts
- Nombre d'électrodes/largeur : 3/0,25 m
- Réglage de l'entrefer : 2 mm
- Vitesse de défilement : 2 m/minute
- Avant que les effets du traitement Corona aient, en partie, disparu, on soude sur une largeur de 2 mm avec une soudeuse manuelle AMIS sur un seul côté le voile et la mousse de polyuréthane hydrophile ; le non tissé absorbant est ensuite inséré entre la mousse et le voile.
- Les trois derniers scellages sont effectués dans les mêmes conditions que précédemment de manière à former un carré de 8 cm x 8 cm, puis les bords du complexe assemblé sont découpés.
- Le support est découpé selon un carré de 15 cm x 15 cm, puis assemblé au complexe précédant par calandrage avec un rouleau de 10 kg, dans deux directions perpendiculaires.
- On procède ensuite à la découpe du pansement final 13 cm x 13 cm, en arrondissant les coins.

- Protocole B : Procédé de traitement d'un pansement à l'oxyde d'éthylène

Les pansements réalisés selon le protocole A, sont introduits dans des chambres climatiques conditionnées à une température oscillant entre 36 et 50°C, préférentiellement de 43°C et d'hygrométrie comprise entre 62 et 80%, préférentiellement de 70%, pendant une durée de 12h. Cette étape de pré-conditionnement permet d'optimiser le temps de séjour en phase active, c'est-à-dire, le temps que passeront les pansements par la suite sous pression d'oxyde d'éthylène. Dans un second temps, les pansements sont transférés dans une autre chambre conditionnée. La seconde chambre présente un vide initial de 70 mbar, avant toute injection préalable de vapeur d'eau, afin d'obtenir un niveau d'hygrométrie de l'ordre de 50%. Par la suite, une première injection d'azote (N2) est réalisée sous une pression partielle de 360 mbars. Dans le même temps, de l'oxyde d'éthylène gazeux est injecté sous une pression partielle de 920 mbars, et enfin une deuxième injection d'azote est réalisée, amenant la pression partielle à une valeur de 930 mbars. La masse d'oxyde d'éthylène est par la suite contrôlée et la température est portée à 45°C pendant une durée d'au moins 6h. La dernière étape consiste alors à effectuer 2 rinçages successifs, en injectant de l'azote sous une pression de 970 mbars, avant de détendre le niveau de pression aux alentours de 150 à 170 mbars.

Protocole C : Mesure du profil de dissolution du principe actif par la méthode du sachet

Le profil de dissolution, ou de relargage, est mesuré conformément à la méthode dite de « méthode du sachet ». Elle consiste à réaliser une analyse sur 3 échantillons de pansements similaires. Ces échantillons présentent une surface de 25 cm$^2$, soit une découpe de 5 cm x 5 cm. Chaque échantillon est préalablement introduit dans un sachet constitué par un non tissé fin, perméable et hydrophobe, puis ces ensembles sont introduits dans un flacon étanche, à plat, l'interface apposée face au fond du récipient. 40 mL de sérum physiologique (H$_2$O + 0,9% de chlorure de sodium) sont introduits dans chacun des flacons du test. Chaque flacon est par la suite fermé et maintenu sous agitation tangentielle à 120 secousses/minutes à 32°C (une sonde de température a été placée dans le thermoshake) pendant 1H, 4H, 7H et 24H. La totalité du surnageant est récupérée à chacune de ces 4 échéances. A chaque échéance, le liquide éliminé a été remplacé par 10 mL de sérum physiologique et chaque flacon est mis sous agitation tangentielle jusqu'à la prochaine échéance. On mesure par chromatographie HPLC (avec détection réfractométrique) la quantité de principe actif présent dans le surnageant à chacune de ces échéances.

II- Exemples de réalisation :

- Exemple n°1 : Pansement selon l'invention

On prépare un pansement en suivant les protocoles A et B.

- Exemple n°2 : Pansement selon l'art antérieur

On prépare un pansement en suivant uniquement le protocole A.

- Résultats :

On applique le protocole d'évaluation C aux deux types de pansements. On obtient des profils de dissolution qui sont reportés sur un même graphe pour permettre la comparaison entre le pansement de l'exemple n°1 et celui de l'exemple n°2.

Les pansements de l'exemple n°1 et de l'exemple n°2 présentent des profils de dissolution tout à fait différents.

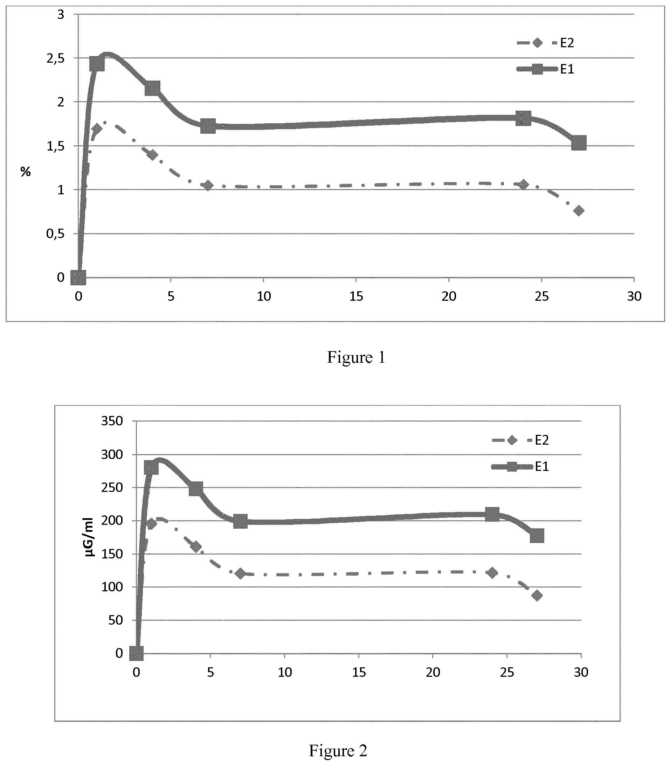

La figure 1 et la figure 2 sont des représentations graphiques rapportant le relargage (valeurs de chaque point de la courbe mesurées de façon non cumulée aux valeurs précédentes) en principe actif de chaque pansement. Les valeurs de relargage sont définies soit en % d'actif relargué en fonction du temps (figure 1), soit en μg/mL d'actif relargé en fonction du temps (figure 2).

Il apparaît de façon très nette que quelle que soit la représentation graphique d'intérêt choisie, le relargage en principe actif est presque deux fois plus élevé pour les pansements traités, objets de la présente invention, que pour les pansements non traités. La conséquence directe de cette caractéristique est que la concentration efficace en principe actif retrouvée sur le site de la lésion est meilleure dans le cas d'un pansement traité à l'oxyde d'éthylène.

Ainsi la résolution des différents problèmes rencontrés au niveau du site de la lésion, tel que les problèmes de cicatrisation, de détersion, de cicatrices pathologiques ou de vergetures, par une concentration efficace supérieure en principe actif retrouvée sur ce site en question, conséquence d'une libération prolongée et supérieure en principe actif, n'en est que meilleure.

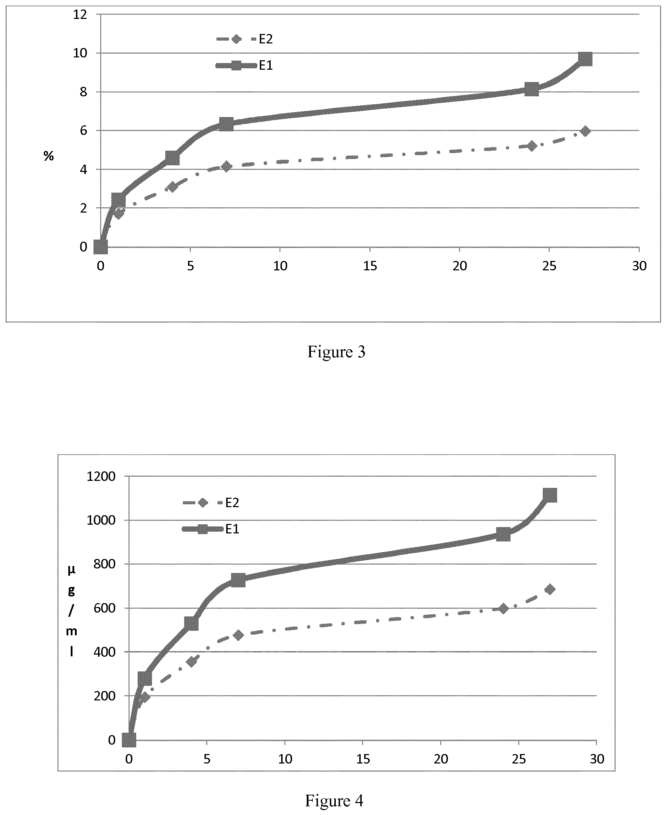

Les figures 3 et 4 reprennent les données des figures 1 et 2 respectivement, mais la représentation des valeurs de relargage est une représentation cumulée (chaque valeur relevée à un point de la courbe est additionnée aux valeurs précédentes). Cette représentation illustre une caractéristique essentielle du produit, la quantité totale relarguée à différents points dans le temps.

Bien entendu, la présente invention n'est pas limitée aux exemples et au mode de réalisation décrits et représentés, mais elle est susceptible de nombreuses variantes accessibles à l'homme de l'art.

**Revendications**

1. Pansement comprenant au moins une interface micro-adhérente, ladite interface micro-adhérente comprenant au moins un composé choisi parmi les oligosaccharides polysulfatés comprenant 1 à 4 oses, leurs sels et leurs complexes, ledit pansement ayant subi un traitement par de l'oxyde d'éthylène.

2. Pansement selon la revendication 1 dans lequel le composé oligosaccharidique polysulfaté est choisi parmi

   - le sel de potassium du sucrose octasulfate,
   - le sel d'argent du sucrose octasulfate,
   - le complexe d'hydroxyaluminium du sucrose octasulfate.

3. Pansement selon la revendication 1 ou la revendication 2 qui comprend de 0,5 à 2mg/cm2, préférentiellement de 0,7 à 1,9 mg/cm2, avantageusement de 0,9 à 1,7mg/cm2 de composé choisi parmi les oligosaccharides polysulfatés de 1 à 4 oses, leurs sels et leurs complexes.

4. Pansement selon l'une quelconque des revendications 1 à 3, dans lequel la structure interface micro-adhérente est une composition adhésive élastomérique.

5. Pansement selon l'une quelconque des revendications précédentes, dans lequel l'interface micro-adhérente comprend :

   - 10 à 60 % en poids d'au moins une résine tackifiante,
   - 2 à 20% en poids, de préférence 12 à 16 % en poids d'au moins un composé hydrocolloïde,
   - 10 à 65 % en poids d'au moins une huile minérale plastifiante,
   - 3 à 25 % en poids d'au moins un polymère élastomérique.

6. Pansement selon l'une quelconque des revendications précédentes, qui comprend de 1 à 15% en poids, et de préférence de 5 à 10% en poids, rapporté au poids total de l'interface micro-adhérente, d'au moins un composé choisi parmi les oligosaccharides polysulfatés de 1 à 4 oses, leurs sels et leurs complexes.

7. Pansement selon l'une quelconque des revendications 1 à 6, comprenant au moins une interface micro-adhérente dans laquelle est incorporé ou sur laquelle est déposé au moins un composé choisi parmi les oligosaccharides polysulfatés comprenant 1 à 4 oses, leurs sels et leurs complexes, présentant une libération prolongée dudit composé, **caractérisée par** une dissolution supérieure ou égale à 4 % en 5h, supérieure ou égale à 5 % en 10h, supérieure ou égale à 5,5 % en 15h, supérieure ou égale à 6 % en 20h, telle que mesurée conformément à la méthode du sachet, dans un milieu de dissolution constitué de 40 mL de sérum physiologique.

8. Pansement selon la revendication 7, qui présente une libération prolongée dudit oligosaccharide, **caractérisée par** une dissolution supérieure ou égale à 5 % en 5h, supérieure ou égale à 5,5 % en 10h, supérieure ou égale à 6 % en 15h, supérieure ou égale à 7 % en 20h, telle que mesurée conformément à la méthode du sachet, dans un milieu de dissolution constitué de 40 mL de sérum physiologique.

9. Pansement selon l'une quelconque des revendications 1 à 8, dans lequel ladite interface micro-adhérente confère au pansement un pouvoir adhésif sur plaque d'acier compris entre 0,5 et 100 cN/cm, et de préférence compris entre 5 et 40 cN/cm.

10. Pansement selon l'une quelconque des revendications 1 à 9, pour son utilisation dans la détersion ou la cicatrisation d'une plaie, dans la prévention et le traitement des vergetures et dans la prévention et le traitement des cicatrices.

11. Procédé de fabrication d'un pansement selon l'une quelconque des revendications 1 à 9 comprenant :

    (a) le dépôt ou l'incorporation, respectivement sur ou dans l'interface micro-adhérente du pansement, d'au moins un composé choisi parmi les oligosaccharides polysulfatés comprenant 1 à 4 unités oses, leurs sels et leurs complexes,
    (b) un traitement par de l'oxyde d'éthylène dudit pansement.

12. Procédé selon la revendication 11 dans lequel l'étape (b) de traitement par de l'oxyde d'éthylène comporte au moins les étapes suivantes :

    (i) une étape de pré-conditionnement,
    (ii) une étape d'exposition à l'oxyde d'éthylène,
    (iii) une étape de rinçage.

13. Procédé selon la revendication 12 dans lequel

    - au cours de l'étape (i) de pré-conditionnement le pansement ou la couche active est soumis à une température allant de 25 à 60°C et un degré d'hygrométrie allant de 50 à 95% pendant une durée allant de 5 à 15h,
    - l'étape (ii) comporte un traitement par de l'oxyde d'éthylène gazeux à une pression supérieure ou égale à 920 mBars dans une enceinte à un degré d'hygrométrie supérieur ou égal à 50, à une température supérieure ou égale à 30°C, et pendant une durée d'au moins 2 heures,
    - l'étape (iii) comporte au moins deux séquences successives d'injection d'azote, suivie d'une détente.

14. Procédé de prévention et/ou de traitement cosmétique des vergetures, ou des cicatrices ou des lésions cutanées dues aux vergetures, ce procédé comprenant l'application sur la ou les zones de la peau concernées d'un pansement selon l'une quelconque des revendications 1 à 9.

15. Procédé de prévention et/ou de traitement cosmétique des cicatrices ou des lésions cutanées, ce procédé comprenant l'application sur la ou les zones de la peau concernées d'un pansement selon l'une quelconque des revendications 1 à 9.

**Patentansprüche**

1. Wunderverband, umfassend mindestens eine mikro-haftende Grenzfläche, wobei die mikro-haftende Grenzfläche mindestens eine Verbindung umfasst, ausgewählt aus Polysulfatoligosacchariden mit 1 bis 4 Osen, ihren Salzen und ihren Komplexen, wobei der Wundverband einer Behandlung mit Ethylenoxid unterzogen wurde.

2. Wundverband nach Anspruch 1, wobei die Polysulfatoligosaccharid-Verbindung ausgewählt ist aus:

- einem Kaliumsalz von Octasulfatsaccharose,
- einem Silbersalz von Octasulfatsaccharose,
- dem Hydroxyaluminiumkomplex von Octasulfatsaccharose.

3. Wundverband nach Anspruch 1 oder Anspruch 2, welcher von 0,5 bis 2 mg/cm$^2$, vorzugsweise von 0,7 bis 1,9 mg/cm$^2$, vorteilhaft von 0,9 bis 1,7 mg/cm$^2$, einer Verbindung umfasst, ausgewählt aus Polysulfatoligosacchariden mit 1 bis 4 Osen, ihren Salzen und ihren Komplexen.

4. Wundverband nach einem der Ansprüche 1 bis 3, wobei die mikro-haftende Grenzflächenstruktur eine haftende elastomere Zusammensetzung ist.

5. Wundverband nach einem der vorhergehenden Ansprüche, wobei die mikro-haftende Grenzfläche umfasst:

- 10 bis 60 Gew.-% mindestens eines klebrigmachenden Harzes,
- 2 bis 20 Gew.-%, vorzugsweise 12 bis 16 Gew.-%, mindestens einer Hydrokolloid-Verbindung,
- 10 bis 65 Gew.-% mindestens eines Weichmachermineralöls,
- 3 bis 25 Gew.-% mindestens eines elastomeren Polymers.

6. Wundverband nach einem der vorhergehenden Ansprüche, welcher von 1 bis 15 Gew.-% und vorzugsweise von 5 bis 10 Gew.-%, im Verhältnis zum Gesamtgewicht der mikro-haftenden Grenzfläche, mindestens einer Verbindung umfasst, ausgewählt aus Polysulfatoligosacchariden mit 1 bis 4 Osen, ihren Salzen und ihren Komplexen.

7. Wundverband nach einem der Ansprüche 1 bis 6, umfassend mindestens eine mikro-haftende Grenzfläche, in der enthalten ist oder auf der abgeschieden ist mindestens eine Verbindung, ausgewählt aus Polysulfatoligosacchariden mit 1 bis 4 Osen, ihren Salzen und ihren Komplexen, mit einer gesteuerten Freisetzung der Verbindung, **gekennzeichnet durch** ein Lösen von größer oder gleich 4 % in 5 h, größer oder gleich 5 % in 10 h, größer oder gleich 5,5 % in 15 h, größer oder gleich 6 % in 20 h, wie gemäß der Beutelmethode gemessen, in einem Lösungsmedium, das aus 40 ml physiologischem Serum besteht.

8. Wundverband nach Anspruch 7, welcher eine gesteuerte Freisetzung des Oligosaccharids aufweist, **gekennzeichnet durch** ein Lösen von größer oder gleich 5 % in 5 h, größer oder gleich 5,5 % in 10 h, größer oder gleich 6 % in 15 h, größer oder gleich 7 % in 20 h, wie gemäß der Beutelmethode gemessen, in einem Lösungsmedium, das aus 40 ml physiologischem Serum besteht.

9. Wundverband nach einem der Ansprüche 1 bis 8, wobei die mikro-haftende Grenzfläche dem Wundverband ein Haftvermögen auf einer Stahlplatte zwischen 0,5 und 100 cN/cm und vorzugsweise zwischen 5 und 40 cN/cm verleiht.

10. Wundverband nach einem der Ansprüche 1 bis 9, zu dessen Verwendung bei der Reinigung oder der Heilung einer Wunde, bei der Prävention und der Behandlung von Dehnungsstreifen und bei der Prävention und der Behandlung von Narben.

11. Verfahren zur Herstellung eines Wundverbands nach einem der Ansprüche 1 bis 9, umfassend:

(a) jeweils Abscheidung auf oder Einschluss in der mikro-haftenden Grenzfläche des Verbands, mindestens einer Verbindung, ausgewählt aus Polysulfatoligosacchariden mit 1 bis 4 Osen, ihren Salzen und ihren Komplexen,
(b) eine Behandlung des Wundverbands mit Ethylenoxid.

12. Verfahren nach Anspruch 11, wobei der Schritt (b) der Behandlung mit Ethylenoxid mindestens die folgenden Schritte umfasst:

(i) einen Schritt der Vorkonditionierung,
(ii) einen Schritt des Aussetzens an Ethylenoxid,
(iii) einen Schritt des Spülens.

13. Verfahren nach Anspruch 12, wobei:

- während des Schritts (i) der Vorkonditionierung der Wundverband oder die aktive Schicht einer Temperatur von 25 bis 60°C und einem Hygrometriegrad von 50 bis 95 % während einer Dauer von 5 bis 15 h ausgesetzt wird,
- der Schritt (ii) eine Behandlung mit gasförmigem Ethylenoxid bei einem Druck größer oder gleich 920 mbar in einer Hülle mit einem Hygrometriegrad größer oder gleich 50 bei einer Temperatur größer oder gleich 30°C und während einer Dauer von mindestens 2 Stunden umfasst,
- der Schritt (iii) mindestens zwei aufeinanderfolgende Sequenzen des Einbringens von Stickstoff, gefolgt von einer Entspannung, umfasst.

14. Verfahren zur Prävention und/oder kosmetischen Behandlung von Dehnungsstreifen oder Narben oder durch die Dehnungsstreifen bedingten Hautläsionen, wobei dieses Verfahren das Aufbringen eines Wundverbands nach einem der Ansprüche 1 bis 9 auf die betroffene Zone oder die betroffenen Zonen der Haut umfasst.

15. Verfahren zur Prävention und/oder kosmetischen Behandlung von Narben oder Hautläsionen, wobei dieses Verfahren das Aufbringen eines Wundverbands nach einem der Ansprüche 1 bis 9 auf die betroffene Zone oder die betroffenen Zonen der Haut umfasst.

**Claims**

1. A dressing comprising at least one micro-adherent interface, the said micro-adherent interface comprising at least one compound selected from among polysulfated oligosaccharides comprising 1 to 4 oses, the salts and complexes thereof, the said dressing having been subjected to treatment with ethylene oxide.

2. The dressing according to claim 1 wherein the polysulfated oligosaccharide compound is selected from among:

   - the potassium salt of sucrose octasulfate,
   - the silver salt of sucrose octasulfate,
   - the hydroxyaluminium complex of sucrose octasulfate.

3. The dressing according to claim 1 or claim 2 which comprises from 0.5 to 2mg/cm$^2$, preferably from 0.7 to 1.9 mg/cm$^2$, advantageously from 0.9 to 1.7mg/cm$^2$ of compound selected from among polysulfated oligosaccharides having 1 to 4 oses, the salts and complexes thereof.

4. The dressing according to any of claims 1 to 3, wherein the micro-adherent interface structure is an adhesive elastomeric composition.

5. The dressing according to any of the preceding claims, wherein the micro-adherent interface comprises:

   - 10 to 60 % by weight of at least one tackifying resin;
   - 2 to 20% by weight, preferably 12 to 16 % by weight of at least one hydrocolloid compound;
   - 10 to 65 % by weight of at least one plasticizing mineral oil;
   - 3 to 25 % by weight of at least one elastomeric polymer.

6. The dressing according to any of the preceding claims, which comprises from 1 to 15 % by weight, preferably from 5 to 10 % by weight relative to the total weight of the micro-adherent interface, of at least one compound selected from among polysulfated oligosaccharides having 1 to 4 oses, the salts and complexes thereof.

7. A dressing according to any one of claims 1 to 6, comprising at least one micro-adherent interface, in which there is incorporated or on which there is deposited at least one compound selected from among polysulfated oligosaccharides having 1 to 4 oses, the salts and complexes thereof, having sustained release of the said compound **characterized by** a dissolution higher or equal to 4 % in 5h, higher or equal to 5 % in 10 h, higher or equal to 5.5 % in 15h, higher or equal to 6% in 20h, such as measured in accordance with the sachet method, in a dissolution medium formed of 40 mL physiological saline.

8. The dressing according to claim 7 having sustained release of the said polysulfated oligosaccharide, **characterized by** a dissolution higher or equal to 5 % in 5h, higher or equal to 5.5 % in 10h, higher or equal to 6 % in 15h, higher or equal to 7 % in 20h, such as measured using the sachet method, in a dissolution medium formed of 40 mL physiological saline.

9. The dressing according to any of claims 1 to 8, wherein the said micro-adherent interface imparts an adhesive power to the dressing measured on a steel plate comprised between 0.5 and 100 cN/cm, and preferably between 5 and 40 cN/cm.

10. The dressing according to any of claims 1 to 9, for use thereof for the debridement or the healing of a wound, in the prevention and the treatment of stretch marks and in the prevention and the treatment of scars.

11. A method for manufacturing a dressing according to any of claims 1 to 9 comprising:

   (a) the depositing or incorporation, respectively on or in the micro-adherent interface of a dressing, of at least one compound selected from among polysulfated oligosaccharides having 1 to 4 ose units, the salts and complexes thereof;
   (b) a treatment of the said dressing with ethylene oxide.

12. The method according to claim 11 wherein step (b) of treatment with ethylene oxide treatment comprises at least the following steps:

   (i) a pre-conditioning step ;

(ii) an exposure step to ethylene oxide;
(iii) a rinsing step.

**13.** The method according to claim 12 wherein:

- at the pre-conditioning step (i) the dressing or the active layer is subjected to a temperature ranging from 25 to 60°C and relative humidity from 50 to 95 % for a time from 5 to 15h;
- step (ii) comprises treatment with gaseous ethylene oxide at a pressure higher or equal to 920 mBars in an chamber having relative humidity higher or equal to 50 % at a temperature higher or equal to 30°C for a time of at least 2 hours;
- step (iii) comprises at least two successive sequences of nitrogen injection followed by an expansion.

**14.** A method for the prevention and/or cosmetic treatment of stretch marks, or scars or skin lesions due to stretch marks, this method comprising the application on the skin area(s) concerned of a dressing according to any of claims 1 to 9.

**15.** A method for the prevention and/or cosmetic treatment of scars or skin lesions, this method comprising the application on the skin area(s) concerned of a dressing according to any of claims 1 to 9.

Figure 1

Figure 2

Figure 3

Figure 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2956322
- EP 230023 A
- WO 8905645 A
- WO 9822114 A
- FR 2824474
- FR 2953522
- WO 2006007844 A
- FR 2916356
- WO 9530394 A
- WO 9410954 A
- WO 2007025546 A
- WO 200708531 A
- WO 9312275 A
- WO 0001425 A
- WO 9416746 A
- WO 9519795 A
- EP 878204 A
- EP 1435247 A
- WO 8601400 A
- US 4102340 A
- EP 358412 A
- US 6096942 A
- FR 1156431
- FR 1156436

**Littérature non-brevet citée dans la description**

- **RICHARD J. ROLANDO.** *Tappi Journal,* Juin 1993, vol. 76 (6
- **MICHAEL T. K. LING et al.** *Antec,* vol. 200, 2724-2730